# EUROPEAN PATENT APPLICATION

(11) **EP 1 932 515 A1**
(43) Date of publication of application: **18.06.2008**
(21) Application number: 06301237.1
(22) Date of filing: 12.12.2006
(51) Int. Cl.: A61K 8/81, A61Q 17/04, A61Q 19/00, C08F 20/54

(54) **Inverse latex of anionic polyelectrolytes in silicone oils; cosmetic use thereof**

(71) Applicant: SOCIETE D'EXPLOITATION DE PRODUITS POUR LES INDUSTRIES CHIMIQUES, S.E.P.P.I.C., 75007 Paris (FR)
(72) Inventor: Braun, Olivier, 81100, CASTRES (FR); Mallo, Paul, 78290, CROISSY- SUR- SEINE (FR)
(74) Representative: Conan, Philippe Claude

(57) **Abstract**

Composition comprising an oil phase, an aqueous phase, at least one emulsifying system of water-in-oil (W/O) type, optionally at least one emulsifying system of oil-in-water (O/W) type, in the form of an inverse latex comprising from 10% to 80% by mass and preferably from 20% to 70% by mass and most preferably from 30% to 60% by mass, of a linear partially or totally salified anionic polyelectrolyte, with wherein in that said oil phase comprises a silicone oil or a mixture of silicone oils; its use to thicken and or to emulsify cosmetic, dermopharmaceutical or pharmaceutical compositions.

## Description

The present patent application relates to water-in-oil inverse latex, to a process for preparing them and to their use as thickeners and/or emulsifiers for skincare and haircare products or for the manufacture of cosmetic, dermocosmetic, dermopharmaceutical or pharmaceutical preparations.

The thickeners used in the cosmetics or pharmaceutical industry, are intended to thicken aqueous phases, lotions, oil-in-water emulsions, water-in-oil emulsions or cream-gels, which are oil-in-water emulsions stabilized with thickeners and without the use of emulsifiers. In the case of oil-in-water emulsions, an emulsifier is also added, especially when it is desired to incorporate a high content of oil into the formulation. However, emulsifiers are often products with a low molecular weight, which are potentially less tolerated by the skin than polymers. Furthermore, the use of polymers allows the preparation of cream-gels without heating, which reduces the manufacturing costs while at the same time keeping the heat-sensitive molecules intact.

Accordingly, it has been sought to develop polymers that are both thickeners and emulsifiers. Synthetic thickening polymers in the form of inverse latex are frequently used, for instance those described in the French patent applications published under the numbers 2 721 511, 2 733 805, 2 774 688, 2 774 996 and 2 782 086 and also in the European patent application published under the number EP 0 503 853.

In most of the inverse latex used in these industries, the oil phase generally comprises a commercial mineral oil containing saturated hydrocarbons, such as paraffins, isoparaffins or cycloparaffins, for instance Marcol^{™} 52, Isopar^{™} M or Isopar^{™} L, isohexadecane, isododecane, synthetic oil such as hydrogenated polydecene or hydrogenated polyisobutene, or a plant oil, for instance squalane or a mixture of several of these oils. Such an oil phase can be considered as a vector, which allows an easier use of the polymer, which itself is the real thickener. After having introduced the thickening composition, and after the swelling of the polymers having been achieved in the formulation in the oil phase of the inverse latex is no more useful.

In some specific area of the cosmetic industry, emulsions with silicone oils have been developed and commercialized. US patent 5,470,551 discloses the use of aqueous dispersion based on organopolysiloxanes and on a crosslinked acrylamide/neutralized 2-acrylamido-2-methylpropanesulphonic acid copolymer for hair treatment.

International application WO 2005/079965 discloses inverse emulsions of cross-linked copolymers containing ionic moieties in silicone oil.

However the inverse latex according to WO 2005/079965, generates emulsions, involving the thickening of aqueous phases or hydro-alcoholic phases, which are not stable enough in the presence of electrolytes containing media such as organic or inorganic UV filters, and which are characterized by a viscosity decrease along the time until a final phase displacement occurs.

Moreover the above-mentioned inverse latex based on silicone oil reduce the so-called "pick-up" effect that is detrimental to a good prehension and a good stiffness of the final emulsion which does not allow a fluid flooding of the cosmetic emulsion from the bottle.

In the context of its research to develop novel emulsifying and thickening compounds, the inventors became interested in novel inverse latex based on silicone oil, which do not induce the above-mentioned effects.

According to a first embodiment, the invention relates to a composition comprising an oil phase, an aqueous phase, at least one emulsifying system of water-in-oil (W/O) type, optionally at least one emulsifying system of oil-in-water (O/W) type, in the form of an inverse latex comprising from 10% to 80% by weight and preferably from 20% to 70% by weight and most preferably from 30% to 60% by weight, of a linear partially or totally salified anionic polyelectrolyte, wherein said oil phase comprises a silicone oil or a mixture of silicone oils.

In the composition as defined above, the words "silicone oil" mean all available cosmetically acceptable silicone oils, and more particularly the silicone oils that are listed in the US and the European Pharmacopoeia. Examples of silicone oils are:

Dimethylpolysiloxanes, dimethylsiloxanes, methylphenylpolysiloxanes, phenyldimethylsiloxanes, cyclopentasiloxanes, silicones modified with amines, silicones modified with fatty acids, silicones modified with alcohols, silicones modified with alcohols and fatty acids, silicones modified with polyether groups, epoxy-modified silicones, silicones modified with fluoro groups, cyclic silicones and silicones modified with alkyl groups.

According to a first more specific embodiment, the present invention relates to a composition as here before defined, wherein the silicone oil is chosen from Dimethicone 5cps, Dimethicone 6cps, Cyclopentasiloxane, or a mixture thereof. Such oils are commercially available under the trade names DM-FLUID-5cs, DC-200-5cst, KF-96-A-5cs, DM-FLUID-6cs, KF-96-A-6cs, Wacker-Belsil CM 040, or KF-995.

The composition as defined above generally comprises between 10% by mass of 50% by mass of oil phase.

In the composition as defined above, the word linear means that the polyelectrolyte is not a cross-linked polyelectrolyte.

In the composition as defined above, the words anionic polyelectrolyte means that the polyelectrolyte contains acid functions, which may be free acid functions, partially salified acid functions or totally salified acid functions. In the context of the invention, acid function means either strong acid function or weak acid function.

According to a second more specific embodiment of the present invention, the anionic polyelectrolyte as defined above, is a copolymer of at least one monomer having a strong acid function either, free, partially salified or totally salified with at least one neutral monomer or a copolymer of at least one monomer having at least a weak acid function either, free, partially salified or totally salified with at least one neutral monomer.

In the context of the present invention, " monomer having a strong acid function" more specifically means a monomer having a sulfonic group (SO₃H), such as, for example, styrenesulfonic acid, vinyl sulfonic acid 2-propenyl sulfonic acid or 2-methyl-2-[(1-oxo-2-propenyl)amino]-1-propanesulfonic acid.

According to a still more specific embodiment of the present invention, the anionic polyelectrolyte as defined above, is a copolymer of 2-methyl-2-[(1-oxo-2-propenyl)amino]-1-propanesulfonic acid, free, partially salified or totally salified with a neutral monomer or a copolymer of a monomer having a weak acid function either, free, partially salified or totally salified with a neutral monomer.

In the context of the present invention, " monomer having a weak acid function" more specifically means a monomer having a carboxylic group (CO₂H), such as, for example, acrylic acid, methacrylic acid, itaconic acid, maleic acid or 3-methyl-3-[(1-oxo-2-propenyl)amino] butanoic acid.

According to another still more specific embodiment of the present invention, the anionic polyelectrolyte as defined above, is a copolymer of acrylic acid or of 3-methyl-3-[(1-oxo-2-propenyl)amino] butanoic acid, free, partially salified or totally salified, with a neutral monomer.

In the context of the present invention, neutral monomer mainly means a monomer chosen from acrylamide, methacrylamide, N-alkyl acrylamide, wherein the alkyl group contains between one and four carbon atoms, such as for example, N-methyl acrylamide, N-ethyl acryl amide, N-propyl acrylamide, N-isopropylacrylamide, N-butylacrylamide, N-terbutylacrylamide, N-alkyl methacrylamide, wherein the alkyl group contains between one and four carbon atoms, such as for example, N-methyl methacrylamide, N-ethyl methacrylamide, N-propyl methacrylamide, N-isopropylmethacrylamide, N-butylmethacrylamide, N-tertbutylmethacrylamide, N,N-dialkyl acrylamide, wherein each of the alkyl groups contains between one and four carbon atoms, such as for example, N,N-dimethyl acrylamide, N,N-diethyl acrylamide, N,N-dipropyl acrylamide, N,N-diisopropyl acrylamide, N,N-dibutylacrylamide, N,N-dialkyl methacrylamide, wherein each of the alkyl groups contains between one and four carbon atoms, such as for example, N,N-dimethyl methacrylamide, N,N-diethyl methacrylamide, N,N'-dipropyl methacrylamide, N,N-diisopropyl methacrylamide, N,N dibutylmethacrylamide, diacetoneacrylamide, N-[2-hydroxy-1,1-bis[(hydroxymethyl)ethyl]] propenamide [or tris(hydroxymethyl) acrylamidomethane or N-tris(hydroxymethyl) methylacrylamide also known as THAM], (2-hydroxyethyl) acrylate, (2,3-dihydroxypropyl) acrylate, (2-hydroxyethyl) methacrylate, (2,3-dihydroxypropyl) methacrylate, an ethoxylated derivative with a molecular weight of between 400 and 1000, of each of these esters or vinylpyrrolidone.

According to another still more specific embodiment of the present invention, the anionic polyelectrolyte as defined above, is a copolymer of a monomer having a strong acid function either, free, partially salified or totally salified with N-tris(hydroxymethyl) methylacrylamide, (also known as THAM) or a copolymer of a monomer having a weak acid function either, free, partially salified or totally salified with N-tris(hydroxymethyl) methylacrylamide, (also known as THAM). As representative examples of this still more specific embodiment, the composition as defined above, is chosen from:
- an inverse latex of a copolymer of 2-methyl-2- [(1-oxo-2-propenyl)amino]-1-propanesulfonic acid, free, partially salified or totally salified with N-tris(hydroxymethyl) methylacrylamide,
- an inverse latex of a copolymer of acrylic acid free, partially salified or totally salified, with N-tris(hydroxymethyl) methylacrylamide, and
- an inverse latex of a copolymer of 3-methyl-3-[(1-oxo-2-propenyl)amino] butanoic acid, free, partially salified or totally salified, with N-tris(hydroxymethyl) methylacrylamide.

According to a third more specific embodiment of the present invention, the anionic polyelectrolyte as defined above, is a terpolymer of at least one monomer having a strong acid function either, free, partially salified or totally salified with at least one first neutral monomer different from N-tris(hydroxymethyl) methylacrylamide, and with N-tris(hydroxymethyl) methylacrylamide.

As representative examples of this still more specific embodiment, the composition as defined above, is chosen from:
- an inverse latex of a terpolymer of 2-methyl-2-[(1-oxo-2-propenyl)amino]-1-propanesulfonic acid, free, partially salified or totally salified, with (2-hydroxyethyl)acrylate and with N-tris(hydroxymethyl) methylacrylamide,
- an inverse latex of a terpolymer of 2-methyl-2-[(1-oxo-2-propenyl)amino]-1-propanesulfonic acid, free, partially salified or totally salified with acrylamide and with N-tris(hydroxymethyl) methylacrylamide, and
- an inverse latex of a terpolymer of 2-methyl-2-[(1-oxo-2-propenyl)amino]-1-propanesulfonic acid, free, partially salified or totally salified with N,N-dimethylacrylamide and with N-tris(hydroxymethyl) methylacrylamide,

According to a fourth more specific embodiment of the present invention, the anionic polyelectrolyte as defined above, is a terpolymer of at least one monomer having a weak acid function either, free, partially salified or totally salified with at least one first neutral monomer different from N-tris(hydroxymethyl) methylacrylamide, and with N-tris(hydroxymethyl) methylacrylamide.

As representative examples of this still more specific embodiment, the composition as defined above, is chosen from:
- an inverse latex of a terpolymer of acrylic acid free, partially salified or totally salified with (2-hydroxyethyl) acrylate and with N-tris(hydroxymethyl) methylacrylamide,
- an inverse latex of a terpolymer of 3-methyl-3-[(1-oxo-2-propenyl)amino] butanoic acid, free, partially salified or totally salified, with (2-hydroxyethyl)acrylate and with N-tris(hydroxymethyl) methylacrylamide,
- an inverse latex of a terpolymer of acrylic acid free, partially salified or totally salified, with acrylamide and with N-tris(hydroxymethyl) methylacrylamide,
- an inverse latex of a terpolymer of 3-methyl-3-[(1-oxo-2-propenyl)amino] butanoic acid, free, partially salified or totally salified, with acrylamide and with N-tris(hydroxymethyl) methylacrylamide,
- an inverse latex of a terpolymer of acrylic acid free, partially salified or totally salified, with N,N-dimethylacrylamide and with N-tris(hydroxymethyl) methylacrylamide, and
- an inverse latex of a terpolymer of 3-methyl-3-[(1-oxo-2-propenyl)amino] butanoic acid, free, partially salified or totally salified, with N,N-dimethylacrylamide and with N-tris(hydroxymethyl) methylacrylamide,

According to a fifth more specific embodiment of the present invention, the anionic polyelectrolyte as defined above, is a terpolymer of at least one monomer having a strong acid function either, free, partially salified or totally salified with at least one monomer having a weak acid function either, free, partially salified or totally salified and with N-tris(hydroxymethyl) methylacrylamide.

As representative examples of this still more specific embodiment, the composition as defined above, is chosen from:
- an inverse latex of a terpolymer of 2-methyl-2-[(1-oxo-2-propenyl)amino]-1-propanesulfonic acid, free, partially salified or totally salified, with acrylic acid free, partially salified or totally salified, and with N-tris(hydroxymethyl) methylacrylamide, and
- an inverse latex of a terpolymer of 2-methyl-2-[(1-oxo-2-propenyl)amino]-1-propanesulfonic acid, free, partially salified or totally salified, with 3-methyl-3-[(1-oxo-2-propenyl)amino] butanoic acid, free, partially salified or totally salified, and with N-tris(hydroxymethyl) methylacrylamide.

In the context of the invention, when the weak and/or the strong acid function are partially or totally salified, said acid functions are mainly salified in the form of either an alkali metal salt, for example the sodium salt or the potassium salt, or the form of the ammonium salt, an amino alcohol salt, for instance the monoethanolamine salt, or an amino acid salt, for instance the lysine salt.

In the context of the invention, if the anionic polyelectrolyte, which is the component of the composition as defined above, is a polymer of at least one monomer having a free, partially or totally salified strong acid function , the molar proportion of said monomeric moiety in said polymer is between 5% and 95 % more specifically between 10% and 90% and still more specifically between 25% and 80%.

In the context of the invention, if the anionic polyelectrolyte, which is the component of the composition as defined above, is a polymer of at least one monomer having a free, partially or totally salified weak acid function, the molar proportion of said monomeric moiety in said polymer is between 5% and 95% more specifically between 10% and 90% and still more specifically between 25% and 80%.

In the context of the invention, if the anionic polyelectrolyte, which is the component of the composition as defined above, is a polymer of at least one neutral monomer, the molar proportion of said monomeric moiety in said polymer is between 5% and 50% more specifically between 10% and 45% and still more specifically between 20% and 40%.

In the context of the invention, if the anionic polyelectrolyte, which is the component of the composition as defined above, is a polymer of N-tris(hydroxymethyl) methylacrylamide, the molar proportion of said N-tris(hydroxymethyl) methylacrylamide moiety in said polymer is between 5% and 50% more specifically between 10% and 40% and still more specifically between 15% and 25%.

According to a very specific embodiment of the invention, the composition as defined above is chosen from:
- an inverse latex of a terpolymer of 50 mole% 2-methyl-2-[(1-oxo-2-propenyl)amino]-1-propanesulfonic acid, free, partially salified or totally salified with 30 mole% N,N-dimethylacrylamide and with 20 mole% N-tris(hydroxymethyl) methylacrylamide,
- an inverse latex of a terpolymer of 50 mole% 2-methyl-2-[(1-oxo-2-propenyl)amino]-1-propanesulfonic acid, free, partially salified or totally salified with 35 mole% N,N-dimethylacrylamide and with 15 mole% N-tris(hydroxymethyl) methylacrylamide and
- an inverse latex of a terpolymer of 50 mole% 2-methyl-2-[(1-oxo-2-propenyl)amino]-1-propanesulfonic acid, free, partially salified or totally salified with 40 mole% N,N-dimethylacrylamide and with 10 mole% N-tris(hydroxymethyl) methylacrylamide.

In the composition as defined above, the emulsifying system of water-in-oil (W/O) type consists either of a sole surfactant or of a mixture of surfactants on condition that the HLB value of the said mixture is low enough to induce water-in-oil emulsions. As emulsifiers of water-in-oil type, there are, for example, sorbitan esters, for instance sorbitan oleate, for instance the product sold by the company SEPPIC under the name Montane^{™} 80, sorbitan isostearate, for instance the product sold by the company SEPPIC under the name Montane^{™} 70, or sorbitan sesquioleate, for instance the product sold by the company SEPPIC under the name Montane^{™} 83. There are also certain polyethoxylated sorbitan esters, for example pentaethoxylated sorbitan monooleate, for instance the product sold by the company SEPPIC under the name Montanox^{™} 81 or pentaethoxylated sorbitan isostearate, for instance the product sold under the name Montanox^{™} 71 by the company SEPPIC. There is also a diethoxylated oleocetyl alcohol, for instance the product sold under the name Simulsol^{™} OC 72 by the company SEPPIC, a tetraethoxylated lauryl acrylate, for instance the product sold under the name Blemmer^{™} ALE 200 or polyesters with a molecular weight of between 1000 and 3000, produced from condensation between a poly(isobutenyl)succinic acid or its anhydride and polyethylene glycols, such as Hypermer^{™} 2296 sold by the company Uniqema, or, finally, block copolymers with a molecular weight of between 2500 and 3500, for instance Hypermer^{™} B246 sold by the company Uniqema or Simaline^{™} IE 200 sold by the company SEPPIC. As emulsifiers of water-in-oil type, there are also silicone-based emulsifiers such as for example, PEG-3 Dimethicone sold by the company Shin-Etsu under the name KF-6015, PEG-9 methyl ether Dimethicone sold by the company Shin-Etsu under the name KF-6016, or PEG-10 Dimethicone sold by the company Shin-Etsu under the name KF-6017.

The composition that is the subject of the present invention generally comprises from 2% to 8% by weight of an emulsifying system of water-in-oil (W/O) type.

When the composition as defined above comprises an emulsifying system of oil-in-water (O/W) type, it consists either of a sole surfactant or of a mixture of surfactants, on condition that the HLB value of the said mixture is high enough to induce oil-in-water emulsions. As emulsifiers of oil-in-water type, there are, for example, ethoxylated sorbitan esters, for instance sorbitan oleate polyethoxylated with 20 moles of ethylene oxide, sold by the company SEPPIC under the name Montanox^{™} 80, sorbitan laurate polyethoxylated with 20 moles of ethylene oxide, sold by the company SEPPIC under the name Montanox^{™} 20, castor oil polyethoxylated with 40 moles of ethylene oxide, sold under the name Simulsol^{™} OL50, decaethoxylated oleodecyl alcohol, sold by the company SEPPIC under the name Simulsol^{™} OC710, heptaethoxylated lauryl alcohol, sold under the name Simulsol^{™} P7 or sorbitan monostearate polyethoxylated with 20 moles of ethylene oxide, sold by the company SEPPIC under the name Montanox^{™} 60. As emulsifiers of oil-in-water type, there are also silicone-based emulsifiers such as for example, PEG-11 Methyl Ether Dimethicone, sold by the company Shin-Etsu under the name KF-6011 or PEG/PPG-18/18 Dimethicone sold by the company Phoenix under the name PECOSIL^{™} DCF-1818.

When the composition that is the subject of the present invention comprises an emulsifying system of oil-in-water (O/W) type, it generally comprises from 3% to 8% by weight of this system.

According to one particular embodiment of the present invention, the composition as defined above comprises an (O/W) emulsifying system.

According to another particular embodiment of the present invention, the composition as defined above comprises at least 50% by weight and not more than 70% by weight of polyelectrolyte. In this case, the composition is preferably prepared by performing the following process:
a) an aqueous phase containing the monomers and the possible hydrophilic additives is emulsified in an organic phase containing the surfactant system of water-in-oil (W/O) type, a mixture consisting of the oil intended to be present in the final composition and of a volatile oil, and the possible hydrophobic additives,
b) the polymerization reaction is initiated by introducing a free-radical initiator into the emulsion formed in a), and the reaction is then allowed to proceed, and
c) the reaction medium from step b) is concentrated by distillation until the said volatile oil has been completely removed.

The volatile oils that are suitable for performing the process as defined above are, for example, light isoparaffins containing from 8 to 11 carbon atoms, for instance those sold under the names Isopar^{™} G, Isopar^{™} L, Isopar^{™} H or Isopar^{™} J.

According to one preferred embodiment of the process as defined above, the polymerization reaction is initiated with a redox couple, such as the cumene hydroperoxide/sodium metabisulfite couple, at a temperature of less than or equal to 10°C, and is then performed either quasi-adiabatically up to a temperature of greater than or equal to 40°C and more particularly greater than or equal to 50°C, or by controlling the change of the temperature.

When step c) is completed, the emulsifying system of oil-in-water type is introduced, if desired, at a temperature below 50°C.

When the composition as defined above comprises less than 50% by weight of polyelectrolyte, it is preferably prepared by performing the following process:
a) an aqueous phase containing the monomers and the possible additives is emulsified in an oil phase in the presence of one or more emulsifiers of water-in-oil type,
b) the polymerization reaction is initiated by introducing a free-radical initiator into the emulsion formed in a), and the reaction is then allowed to proceed,
c) when the polymerization reaction is completed, one or more emulsifiers of oil-in-water type are introduced, if desired, at a temperature below 50°C.
   According to one variant of this process, the reaction medium obtained form step b) is concentrated by distillation before performing step c).

According to one preferred embodiment of the process as defined above, the polymerization reaction is initiated with a redox couple, such as the cumene hydroperoxide/sodium metabisulfite couple, at a temperature of less than or equal to 10°C, and is then performed either quasi-adiabatically up to a temperature of greater than or equal to 40°C and more particularly greater than or equal to 50°C, or by controlling the change of the temperature.

According to another particular embodiment of the present invention, the composition as defined above comprises not more than 30% by weight of polyelectrolyte.

According to another embodiment, the invention relates to a cosmetic, dermopharmaceutical or pharmaceutical composition, characterized in that it comprises as thickening and/or emulsifying compound, at least one inverse latex as defined above.

The cosmetic, dermocosmetic, dermopharmaceutical or pharmaceutical composition defined above generally comprises from 0.1% to 10% and more particularly between 0.5% and 5% by weight of the said inverse latex. It is especially in the form of a milk, a lotion, a gel, a cream-gel, a water in oil emulsion, an oil in water emulsion, a cream, a soap, a bubblebath, a balm, a shampoo or a conditioner.

According to one preferred embodiment of the present invention, the cosmetic, dermocosmetic, dermopharmaceutical or pharmaceutical composition as defined above is a topical composition.

A subject of the invention is also the use of the inverse latex as defined above for proposing a cosmetic, dermopharmaceutical or pharmaceutical topical composition.

The topical composition according to the invention, intended to be applied to the skin, to the scalp, or to mucous membranes of man or animals, may consist of a topical emulsion comprising at least one aqueous phase and at least one oil phase. This topical emulsion may be of the oil-in-water type. More particularly, this topical emulsion may consist of a fluid emulsion, such as a milk or a fluid gel. The oil phase of the topical emulsion may consist of a mixture of one or more oils.

A topical composition according to the invention may be intended for cosmetic use or may be used to prepare a medicament for treating skin, scalp and mucous membrane diseases. In the latter case, the topical composition then comprises an active principle that may consist, for example, of an antiinflammatory agent, a muscle relaxant, an antifungal agent or an antibacterial agent.

The compositions according to the invention may also contain ingredients usually used in the cosmetic and dermopharmaceutical fields and known to those skilled in the art, such as fats (oils, butters, waxes, fatty acids and gums), emulsifiers and coemulsifiers, gelling agents and/or stabilizers and/or film-forming agents, fillers, pigments, sunscreens, humectants, solvents and cosolvents, plasticizers, sequestrants, antioxidants, fragrances, preserving agents or active principles. As examples of oils that may be combined with the composition of the invention, mention may be made of paraffins, isoparaffins, white mineral oils, plant oils, animal oils, synthetic oils, silicone oils and fluoro oils; and more particularly:
- oils of plant origin, such as sweet almond oil, coconut oil, castor oil, jojoba oil, olive oil, rapeseed oil, groundnut oil, sunflower oil, wheatgerm oil, corn germ oil, soybean oil, cottonseed oil, alfalfa oil, poppyseed oil, pumpkin oil, evening primrose oil, millet oil, barley oil, rye oil, safflower oil, candlenut oil, passionflower oil, hazelnut oil, palm oil, shea butter, apricot kernel oil, beauty-leaf oil, sysymbrium oil, avocado oil, calendula oil and floral or legume oils;
- ethoxylated plant oils;
- oils of animal origin, such as squalene and squalane;
- mineral oils, such as liquid paraffin, liquid petroleum jelly and isoparaffins;
- synthetic oils, especially fatty acid esters such as butyl myristate, propyl myristate, cetyl myristate, isopropyl palmitate, butyl stearate, hexadecyl stearate, isopropyl stearate, octyl stearate, isocetyl stearate, dodecyl oleate, hexyl laurate, propylene glycol dicaprylate, esters derived from lanolic acid, such as isopropyl lanolate or isocetyl lanolate, fatty acid monoglycerides, diglycerides and triglycerides, for instance glyceryl triheptanoate, alkylbenzoates, poly-α-olefins, polyolefins, for instance polyisobutene, synthetic isoalkanes, for instance isohexadecane, isododecane, perfluoro oils and silicone oils. Among the silicone oils, mention may be made more particularly of dimethylpolysiloxanes, dimethylsiloxanes, phenyldimethylsiloxanes, cyclopentasiloxanes, methylphenylpolysiloxanes, silicones modified with amines, silicones modified with fatty acids, silicones modified with alcohols, silicones modified with alcohols and fatty acids, silicones modified with polyether groups, epoxy-modified silicones, silicones modified with fluoro groups, cyclic silicones and silicones modified with alkyl groups.

As another fatty substance that may be combined with the composition of the invention, mention may be made of fatty alcohols or fatty acids.

The fatty phase of the preparations according to the invention may also contain waxes such as beeswax, carnauba wax, candelilla wax, ouricury wax, japan wax, cork fibre wax or sugarcane wax, paraffin waxes, lignite waxes, microcrystalline waxes, lanolin wax, ozokerite, polyethylene wax, hydrogenated oils, silicone waxes, plant waxes, fatty alcohols and fatty acids that are solid at room temperature, glycerides that are solid at room temperature.

The inverse latex according to the invention may optionally be combined with other thickening and/or emulsifying polymers. Examples that may be mentioned include homopolymers or copolymers of acrylic acid or of acrylic acid derivatives, homopolymers or copolymers of acrylamide, homopolymers or copolymers of acrylamide derivatives, homopolymers or copolymers of acrylamidomethylpropanesulfonic acid, of vinyl monomer, of trimethylaminoethyl acrylate chloride sold under the names Carbopol^{™}, Ultrez^{™} 10, Pemulen^{™} TR1, Pemulen^{™} TR2, Simulgel^{™} A, Simulgel^{™} NS, Simulgel^{™} EPG, Simulgel^{™} EG, Luvigel^{™} EM, Salcare^{™} SC91, Salcare^{™} SC92, Salcare^{™} SC95, Salcare^{™} SC96, Flocare^{™} ET100, Hispagel^{™}, Sepigel^{™} 305, Sepigel^{™} 501, Sepigel^{™} 502, Sepiplus^{™} 400, Sepiplus^{™} 265, Sepiplus^{™} 250, Sepinov^{™} EMT 10, Novemer^{™} EC1, Aristoflex^{™} AVC, Aristoflex^{™} HBM, Rapithix^{™} A60, Rapithix^{™} A100, Comedia^{™} SP, Flocare^{™} ET58 and Stabileze^{™} 06; hydrocolloids of plant or biosynthetic origin, for instance xanthan gum, karaya gum, carrageenates or alginates; silicates; cellulose and its derivatives; starch and its hydrophilic derivatives; polyurethanes.

The composition according to the invention is also an advantageous substitute for those sold under the names Sepigel^{™} 305, Sepigel^{™} 501, Simulgel^{™} EG, Simulgel^{™} NS or Simulgel^{™} 600 by the Applicant, since it also shows good compatibility with the other excipients used for the preparation of formulations such as milks, lotions, creams, cream-gels, water-in-oil emulsions, oil-in-water emulsions, soaps, bubblebaths, balms, shampoos or hair conditioners.

It is especially compatible with the concentrates described and claimed in the international publications WO 92/06778, WO 95/04592, WO 95/13863, WO 96/37285, WO 98/22207, and WO 98/47610 or in FR 2 734 496, with the surfactants described in WO 93/08204.

Among the emulsifiers that may be used in the presence of the inverse latex according to the invention, examples that may be mentioned include fatty acids, ethoxylated fatty acids, fatty acid esters of sorbitol, ethoxylated fatty acid esters, polysorbates, polyglycerol esters, ethoxylated fatty alcohols, sucrose esters, alkylpolyglycosides, sulfated or phosphated fatty alcohols, mixtures of alkylpolyglycosides and of fatty alcohols described in French patent applications 2 668 080, 2 734 496, 2 756 195, 2 762 317, 2 784 680, 2 784 904, 2 791 565, 2 790 977, 2 807 435,2 804 432, 2 830 774, 2 830 445, mixtures of emulsifiers chosen among alkylpolyglycoside, mixtures of alkylpolyglycosides and of fatty alcohols, polyglycerol esters, polyol esters, polyglycol esters such as polyglycol polyhydroxystearate described in French patent applications 2 852 257, 2 858 554, 2 820 316 and 2 852 258 for preparing water-in-oil emulsions.

The cosmetic, dermocosmetic, dermopharmaceutical or pharmaceutical composition as defined above may also comprise texture agents and/or fillers, for instance acrylic and methacrylic acid copolymers, starches, silicas, calcium, magnesium, aluminum or barium silicates, calcium phosphate, natural fibers such as cotton fibre, cellulose fibre or chitosan fibre, or synthetic fibers such as polyamide (Nylon^{®}) fibre, rayon fibre, viscose fibre, cellulose acetate fibre, polyp-phenyleneterephthamide fibre (Kevlar^{®}), polyethylene or polypropylene fibre, glass fibre, carbon fibre, Teflon fibre, polyester fibre, polyvinyl chloride fibre, polyvinyl alcohol fibre, polyacrylonitrile fibre, polyurethane fibre or polyethylene phthalate fibre, talc, mica, sericite, silica, boron nitride, lauroyllysine, silicone resin powders, calcium carbonate, magnesium carbonate, titanium oxide, zinc oxide or cerium oxide, titanium micas, iron oxides and other mineral or organic pigments, or a mixture of these powders.

As examples of active principles that may be combined with the inverse latex according to the invention, mention may be made of compounds with a lightening or depigmenting action, a moisturizing action, a tensioning action, a soothing or relaxing action, a slimming action, a lipolytic action, a purifying, seboregulatory or hair-loss-countering action, an anti-ageing action, or a firming, restructuring action, a free-radical-scavenging action, an antioxidant action or a self-tanning action. The composition of the invention may thus be combined with active agents such as, for example, arbutin, kojic acid, hydroquinone, ellagic acid, vitamin C and its derivatives, Stay C, magnesium ascorbyl phosphate and its derivatives, ascorbyl glucoside, phytic acid, fruit acids, rucinol or resorcinol, azeleic acid, dihydroxyacetone (DHA), lipoic acid, Sepiwhite^{™} MSH, Vegewhite^{™}, Gatuline^{™}, Synerlight^{™}, Biowhite^{™}, Phytolight^{™}, Dermalight^{™}, Clariskin^{™}, Melaslow^{™}, Dermawhite^{™}, Ethioline^{™}, Melarest^{™}, Gigawhite^{™}, Albatine^{™}, Lumiskin^{™}, Adiposlim^{™}, Adipoless^{™}, polyphenol extracts, grape extracts, pine extracts, wine extracts, olive extracts, tea extracts, cocoa extracts, Amazonian forest plant extracts, legume extracts, floral extracts, fruit extracts, mint extracts, pond extracts, N-acyl proteins, N-acyl peptides, for instance Matrixyl^{™}, N-acylamino acids, partial hydrolysates of N-acyl proteins, amino acids, peptides, total protein hydrolysates, partial protein hydrolysates, polyols (for instance glycerol, butylene glycol, etc.), milk derivatives, Aquaxyl^{™}, urea, pyrrolidonecarboxylic acid or derivatives of this acid, glycyrrhetinic acid or its derivatives, α-bisabolol, sugars or sugar derivatives, polysaccharides or derivatives thereof, hydroxy acids, for instance lactic acid or salicylic acid, vitamins, vitamin derivatives, for instance retinol, retinol derivatives, vitamin E and its derivatives, minerals, trace elements, extracts of rocks or stones, enzymes or derivatives thereof, coenzymes or derivatives thereof, for instance coenzyme Q10, hormones or "hormone-like" substances, for instance Phyto age^{™}, soybean extracts, for instance Raffermine^{™}, wheat extracts, for instance Tensine^{™} or Gliadine^{™}, plant extracts, tannin-rich plant extracts, isoflavone-rich extracts or terpene-rich extracts, freshwater or saltwater algal extracts, marine extracts in general, including coral extracts, essential waxes, bacterial extracts, minerals, for instance the range of Givobio^{™} products and especially the calcium, magnesium, copper, cobalt, zinc, manganese, etc. derivatives, lipids in general, lipids such as ceramides or phospholipids and also derivatives, active agents with a slimming action, for instance caffeine or its derivatives, active agents that improve the capillary circulation of the skin, for instance venotonic agents, draining active agents, decongestive active agents such as ginko biloba, ivy, common horsechestnut, bamboo, ruscus, centella asiatica, fucus, rosemary or sage, active agents with antimicrobial activity or a purifying action on greasy skin, for instance copper or zinc derivatives or octopirox or Sensiva SC50, active agents with energizing or stimulating properties, for instance Sepitonic^{™} M3 or Physiogenyl^{™}, panthenol and its derivatives, for instance Sepicap^{™} MP, anti-ageing active agents, Sepivinol^{™}, Sepivital^{™}, Manoliva^{™} and Phyto age^{™}. The composition of the invention may also more generally be combined with anti-ageing active agents for combating photo-ageing, the targeted active agents protecting the integrity of the dermo-epidermal junction, active agents that increase the synthesis of components of the extracellular matrix (for instance collagen, elastins, glycosaminoglycans, etc.), active agents that act favorably on chemical (cytokines) or physical (integrins) cell communication, active agents with a restructuring effect, active agents with a cicatrizing effect, active agents with a firming effect, active agents with a "botox-like" effect, active agents that act on expression wrinkles, active agents that act on the calcium channels, active agents that improve the integrity of the skin barrier, active agents that act on specific skin enzymes, active agents that act on specific cell receptors, active agents that improve cell communication, active agents with a free-radical-scavenging or anti-oxidant effect, active agents with a "tensioning" effect and active agents with an antidandruff, anti-acne, calmative or anti-neuromediator effect. The composition containing the polymer according to the invention may also be combined with active agents that afford a heating effect on the skin, such as skin capillary circulation activators (for example nicotinates) or ingredients that create, conversely, a sensation of freshness on application (for example menthol).

As sunscreens that may be incorporated with the composition of the invention, mention may be made of any of those featured in the amended Cosmetic Directive 76/768/EEC appendix VII.

According to this preferred embodiment, the sunscreen is more particularly chosen from lipophilic sunscreens, for instance octocrylene, etocrylene, homosalate, for instance Eusolex^{™} HMS, octyl para-methoxycinnamate, for instance Parsol^{™} MCX, octinoxate, octisalate, avobenzone, oxybenzone, benzophenone-1, benzophenone-2, benzophenone-3, for instance Uvinul M-40, benzophenone-8, benzophenone-12, ethyl dihydroxypropyl PABA, glyceryl PABA, ethylhexyl dimethyl PABA, menthyl anthranilate, methylbenzylidenecamphor or isopropyldibenzoylmethane.

The sunscreen as defined above may also comprise one or more lipophobic sunscreens, for instance titanium dioxide, zinc oxide, phenylbenzimidazolesulfonic acid, benzophenone-4, TEA salicylate, PABA and DEA methoxycinnamate.

The sunscreen as defined above may also comprise one or more oil absorbers, for instance silica, whether these are spherical silicas, for instance Spheron^{™} L-1500, porous silica or pyrogenic silica, crosslinked or non-crosslinked polymethyl methacrylate, for instance the Micropearl^{™} products, dextrins, cyclodextrins, molecular sieves, for instance zeolites, Nylon 6 or 12, sodium calcium alumino silicate, talc or mica.

The sunscreen as defined above may also comprise one or more esters of neopentanoic acid with an isoalkyl alcohol containing from 10 to 22 carbon atoms. In this case, it preferably comprises isodecyl neopentanoate, isostearyl neopentanoate or isoarachidyl neopentanoate.

According to a particular embodiment of the invention, the cosmetic, dermopharmaceutical or pharmaceutical composition comprises an efficient quantity of dihydroxyacetone and more particularly between 1% and 8 % by weight of the composition of dihydroxyacetone.

According to a more particular embodiment of the invention, the cosmetic, dermopharmaceutical or pharmaceutical composition comprises either dihydroxyacetone and erythrulose, either dihydroxyacetone and at least a hydroxy acid such as lactic acid, salicylic acid, gluconic acid or kojic acid either dihydroxyacetone and at least one sunscreen agent either dihydroxyacetone and at least one moisturizing agent, either dihydroxyacetone and at least on slimming agent such as caffeine Adiposlim^{™}, Adipoless^{™}.

The examples that follow are intended to illustrate the present invention without, however, limiting it.

### A) - Examples of preparation of compositions according to the invention

### Example 1: Inverse latex of an (AMPS Na salt)/DMA/THAM terpolymer (50/40/10), in polydimethylsiloxane-5cs (Composition 1)

a) - The following are placed in a reactor with stirring:
   - 213 g of deionized water,
   - 354 g of a commercial solution containing 55% sodium 2-methyl-2-[(1-oxo-2-propenyl)amino]-1-propanesulfonate (AMPS Na salt),
   - 67.3 g of N,N-dimethylacrylamide (DMA),
   - 0.5g of sodium diethylenetriaminepentaacetate,
   - 29,8 ofN-tris(hydroxymethyl) methylacrylamide, (THAM)
   The pH of this aqueous solution is equal to 4,0.
b) - An organic phase is prepared by mixing together:
   - 270 g of Polydimethylsiloxane ,characterized by a viscosity of 5 cSt (commercialized under the name DC-200-5cSs by Dow-Corning).
   - 15 g of PEG -10 Dimethicone (commercialized under the name KF-6017 by Shin Etsu)
c) - The aqueous phase is gradually introduced into the organic phase and the mixture is stirred vigorously using a rotor-stator apparatus, commercialized by Silverson^{™} or IKA^{™} . The emulsion obtained is then transferred into a polymerization reactor, purged with nitrogen and then cooled to about 5-6°C. 250 ml of a solution containing 0.635% by weight of cumene hydroperoxide in isohexadecane is then added, followed, after homogenization of the solution, by addition of an aqueous solution of sodium metabisulfite (0.2% by weight in water) at a rate of 0.2 ml/minute for about 60 minutes, while allowing the temperature to rise to room temperature at the end of polymerization. The reaction medium is then maintained for about 90 minutes at this temperature. 4% of PEG-11 Methyl Ether Dimethicone (commercialized under the name KF - 6011 by Shin Etsu) is then added to obtain the desired inverse latex.

### Evaluation of the properties

Viscosity of the inverse latex at 3% by weight in deionized water
(Brookfield RVT spindle 6, speed 5): η = 27 400 mPa.s
Viscosity of the inverse latex at 3% by weight in deionized water + 0.1% NaCl
(Brookfield RVT spindle 3, speed 5): η = 8 400 mPa.s

### Example 2: Inverse latex of an (AMPS Na salt)/DMA/THAM terpolymer (50/35/15), in polydimethylsiloxane-5cs (Composition 2)

The experimental conditions of Example 1 are reproduced, but using 59 g of DMA instead 67.3 g and 44.7 g of THAM instead 29.8g of the above example. The expected inverse latex is obtained.

### Evaluation of the properties

Viscosity of the inverse latex at 3% by weight in deionized water
(Brookfield RVT spindle 6, speed 5): η = 46 400 mPa.s
Viscosity of the inverse latex at 3% by weight in deionized water + 0.1% NaCl
(Brookfield RVT spindle 3, speed 5): η = 9 320 mPa.s

### Example 3: Inverse latex of an (AMPS Na salt)/DMA/THAM terpolymer (50/30/20), in polydimethylsiloxane-5cs (Composition 3)

The experimental conditions of Example 1 are reproduced, but using.50.5 g DMA (instead 67.3g and 59.6 g THAM instead 29.8 g. The expected inverse latex is obtained.

### Evaluation of the properties

Viscosity of the inverse latex at 3% by weight in deionized water
(Brookfield RVT spindle 6, speed 5): η = 56 200 mPa.s
Viscosity of the inverse latex at 3% by weight in deionized water + 0.1% NaCl
(Brookfield RVT spindle 3, speed 5): η = 9 340 mPa.s

The above-mentioned viscosity results unexpectedly shows that le composition according to invention has a thickening and /or an emulsifying effect even if the polyelectrolyte is not cross-linked.

### B) Evaluation of the texture properties

The texture properties of aqueous gels prepared by using compositions according to the invention have been characterized by measuring the adhesion energy, the compression energy and the ratio of the adhesion energy on the compression energy of such aqueous gels.

### B1) Equipment

The adhesion energy and the compression energy of aqueous gels, obtained by the use of compositions according to the invention, have been measured with the help of an apparatus called "texture analyzer". This texture analyzer, commercialized by the company ETIA under the trade-name "TEC", consists of a translation unit on which is mounted a probe, which uses a force sensor for continuous measurement of the stress variations to which the sample is subjected during probe movement.

The movement, its parameters set by the operator, consists of a simple downward movement of the probe into the sample and/or an upward movement out of the sample.

The changing forces are recorded by the ETIA software and represented as a curve of force against time.

### B2) Protocol

### Preparation of the sample:

The aqueous gel is prepared by mixing the composition according to the invention to demineralised water, in a quantity allowing to achieve a viscosity near 50 000 mPa.s. The aqueous gel then obtained is placed at least 24 h before the measurement in a standard container (e.g. inner diameter of 7.7 cm and height of 4.0 cm), the dimensions of which have been designed to enable measurements with all the probes while avoiding edge effects.

### Measurement:

A cycle protocol assesses simultaneously the compression (also designed as the consistency) of the sample and its properties of adhesion to the probe for an aqueous gel.
Such a cycle protocol uses a downward movement of the probe into the sample then an upward movement ; this sequence is repeated once and the two cycles are operated.
For such aqueous gels and such cycle, the probe is hemispherical, the downward movement of the probe is executed at a speed of 1 mm.s-1, the trigger level is of 0,1 Newton, the depth of penetration of the probe into the sample is of 10 mm, and the height of upward movement between each cycle is of 20 mm.
The probe is lowered as close as possible to the sample, and then the cycle is executed by the apparatus by the use the ETIA TEC95 software implemented in a computer connected to the texture analyzer apparatus.
When the measurement has been achieved, the sample surface is flatten by using the back of a spatula. A minimum rest time of 20 min must be observed before proceeding with another measurement on the same sample
As a general rule, the measurements are made in duplicate. In all cases, the measurement is repeated until two measurements are obtained with a difference less than 10%.

### Analysis of the results measured :

During the cycle protocol, the maximum compression force, expressed in Newton, and the minimum traction force, expressed in Newton, are recorded every ten seconds by the apparatus. The software allows the apparatus to draw the compression and the traction charts, and also to calculate the compression energy (Ec), expressed in Joule, and the traction energy considered as the adhesion energy (Ea), expressed in Joule, on the probe.
The sample is considered as having good and efficient prehension properties, or "pick-up" properties, when the ratio between the adhesion energy and the compression energy is higher than a value of 0.4, and more preferentially comprised between a value of 0.70 and 1.5.

### B3) Experimental results measured on aqueous gels prepared by using composition according to the invention

The results, measured according the previous protocol described in B2), on aqueous gels prepared by using compositions according to the invention are contained in the following table :

| **Composition** | **Viscosity after 24 hours** **(mPa.s)** | **Adhesion energy** **(mJoule)** | **Compression energy** **(mJoule)** | **Adhesion energy/ Compression energy** |
|---|---|---|---|---|
| Composition 1 | 48 400 | 1.049 | 2.1 | 0.5 |
| Composition 2 | 51 400 | 1.531 | 2.175 | 0.704 |
| Composition 3 | 49 800 | 1.608 | 2.15 | 0.748 |

The above mentioned ratio of adhesion energy/compression energy results unexpectedly show that the compositions according to the invention allow to prepare aqueous gels which are characterized by good prehension and good stiffness properties even if the polyelectrolyte is not cross-linked.

### C) Examples of formulations prepared with the compositions according to the invention

### Example 4: Care cream

| | | |
|---|---|---|
| Cyclomethicone: | | 10% |
| Inverse latex of Example 1: | | 0.8% |
| Montanov^{™} 68: | | 2% |
| Stearyl alcohol: | | 1% |
| Stearyl alcohol: | | 0.5% |
| Preserving agent: | | 0.65% |
| Lysine: | | 0.025% |
| EDTA (disodium salt): | | 0.05% |
| Xanthan gum: | | 0.2% |
| Glycerol: | | 3% |
| Water: | qs | 100% |

### Example 5: Aftershave balm

### FORMULA

| | | | |
|---|---|---|---|
| A | Inverse latex of Example 3: | | 1.5% |
| | Water: | qs | 100% |
| B | Micropearl^{™} M 100: | | 5.0% |
| | Sepicide^{™} CI: | | 0.50% |
| | Fragrance: | | 0.20% |
| | 95° ethanol: | | 10.0% |

### PROCEDURE

Add B to A.

### Example 6: Non-greasy antisun gel

### FORMULA

| | | | |
|---|---|---|---|
| A | Inverse latex of Example 2: | | 3.00% |
| | Water: | | 30% |
| B | Sepicide^{™} CI: | | 0.20% |
| | Sepicide^{™} HB: | | 0.30% |
| | Fragrance: | | 0.10% |
| C | Dye: | | qs |
| | Water: | | 30% |
| D | Micropearl^{™} M 100: | | 3.00% |
| | Water: | qs | 100% |
| E | Silicone oil: | | 2.0% |
| | Parsol^{™} MCX: | | 5.00% |

### PROCEDURE

Introduce B into A; add C, then D, then E.

### Example 7: Massage gel

### FORMULA

| | | |
|---|---|---|
| A | Inverse latex of Example 3: | 3.5% |
| | Water: | 20.0% |
| B | Dye: | 2 drops/100 g |
| | Water: | qs |
| C | Alcohol: | 10% |
| | Menthol: | 0.10% |
| D | Silicone oil: | 5.0% |

### PROCEDURE

Add B to A, then add C to the mixture, followed by D.

### Example 8: Moisturizing and matting foundation

### FORMULA

| | | | |
|---|---|---|---|
| A | Water: | | 20.0% |
| | Butylene glycol: | | 4.0% |
| | PEG-400: | | 4.0% |
| | Pecosil^{™} PS100: | | 1.0% |
| | NaOH: | qs pH = 9 | |
| | Titanium dioxide: | | 7.0% |
| | Talc: | | 2.0% |
| | Yellow iron oxide: | | 0.8% |
| | Red iron oxide: | | 0.3% |
| | Black iron oxide: | | 0.05% |
| B | Lanol^{™} 99: | | 8.0% |
| | Caprylic/capric triglyceride: | | 8.0% |
| | Montanov^{™} 202: | | 5.00% |
| C | Water: | qs100% | |
| | Micropearl^{™} M305: | | 2.0% |
| | Tetrasodium EDTA: | | 0.05% |
| D | Cyclomethicone: | | 4.0% |
| | Xanthan gum: | | 0.2% |
| | Inverse latex of Example 5: | | 0.8% |
| E | Sepicide^{™} HB: | | 0.5% |
| | Sepicide^{™} CI: | | 0.3% |
| | Fragrance: | | 0.2% |

### PROCEDURE

Prepare mixtures B + D and A + C at 80°C, then mix together and emulsify the whole.

### Example 9: Body milk

### FORMULA

| | | |
|---|---|---|
| Montanov^{™} S: | | 3.5% |
| Lanol^{™} 37T: | | 8.0% |
| Solagum^{™} L: | | 0.05% |
| Water: | qs | 100% |
| Benzophenone-3: | | 2.0% |
| Dimethicone 350 cPs: | | 0.05% |
| Inverse latex of Example 4: | | 0.8% |
| Preserving agent: | | 0.2% |
| Fragrance: | | 0.4% |

### Example 10: Eye contour gel

### FORMULA

| | | |
|---|---|---|
| Inverse latex of Example 2: | | 2.0% |
| Fragrance: | | 0.06% |
| Sodium pyrrolidinonecarboxylate: | | 0.2% |
| Dow Corning^{™} 245 Fluid: | | 2.0% |
| Water: | qs100% | |

### Example 11: Leave-in care composition

### FORMULA

| | | |
|---|---|---|
| Inverse latex of Example 3: | | 1.5% |
| Fragrance: | | qs |
| Preserving agent: | | qs |
| Dow Corning^{™} X2 8360: | | 5.0% |
| Dow Corning^{™} Q2 1401: | | 15.0% |
| Water: | qs100% | |

### Example 12: Ultra-natural tinted cream-gel

### FORMULA

| | | | |
|---|---|---|---|
| A | Water: | | 10.0% |
| | Butylene glycol: | | 4.0% |
| | PEG-400: | | 4.0% |
| | Pecosil^{™} PS100: | | 1.5% |
| | NaOH: | qs pH = 7 | |
| | Titanium dioxide: | | 2.0% |
| | Yellow iron oxide: | | 0.8% |
| | Red iron oxide: | | 0.3% |
| | Black iron oxide: | | 0.05% |
| B | Lanol^{™} 99: | | 4.0% |
| | Caprylic/capric triglyceride: | | 4.0% |
| | Sepifeel^{™} One: | | 1.0% |
| | Inverse latex of Example 5: | | 3.0% |
| C | Water: | qs 100% | |
| | Micropearl^{™} M305: | | 2.0% |
| | Tetrasodium EDTA: | | 0.05% |
| | Cyclomethicone: | | 4.0% |
| D | Sepicide^{™} HB: | | 0.5% |
| | Sepicide^{™} CI: | | 0.3% |
| | Fragrance: | | 0.2% |

### PROCEDURE

Prepare the mixture B + C, then add A and then D.

### Example 13: Non-greasy self-tanning product for the face and body

### FORMULA

| | | | |
|---|---|---|---|
| A | Lanol^{™} 2681: | | 3.0% |
| | Inverse latex of Example 3: | | 2.5% |
| B | Water: | qs 100% | |
| | Dihydroxyacetone: | | 3.0% |
| C | Fragrance: | | 0.2% |
| | Sepicide^{™} HB: | | 0.8% |
| | NaOH (sodium hydroxide): | qs | pH = 5 |

### Example 14: Antisun care product for the face

### FORMULA

| | | | |
|---|---|---|---|
| A | Cyclomethicone and dimethiconol: | | 4.0% |
| | Inverse latex of Example 2: | | 3.5% |
| B | Water: | qs 100% | |
| C | Fragrance: | | 0.1 % |
| | Sepicide^{™} HB: | | 0.3% |
| | Sepicide^{™} CI: | | 0.21% |
| | Parsol^{™} MCX: | | 5.0% |
| | Titanium mica: | | 2.0% |
| | Lactic acid: | qs pH = 6.5 | |

### Example 15: Self-tanning emulsion

### FORMULA

| | | | |
|---|---|---|---|
| A | Lanol^{™} 99: | | 15.0% |
| | Montanov^{™} 68: | | 5.0% |
| | Parsol^{™} MCX: | | 3.0% |
| B | Water: | qs 100% | |
| | Dihydroxyacetone: | | 5.0% |
| | Monosodium phosphate: | | 0.2% |
| C | Inverse latex of Example 1: | | 0.5% |
| D | Fragrance: | | 0.3% |
| | Sepicide^{™} HB: | | 0.8% |
| | NaOH: | qs pH=5 | |

### Example 16: Care cream

| | | |
|---|---|---|
| Cyclomethicone: | | 10.0% |
| Inverse latex of Example 2: | | 0.8% |
| Montanov^{™} 68: | | 4.5% |
| Preserving agent: | | 0.65% |
| Lysine: | | 0.025% |
| EDTA (disodium salt): | | 0.05% |
| Xanthan gum: | | 0.2% |
| Glycerol: | | 3.0% |
| Water: | qs 100% | |

### Example 17: Care cream

| | | |
|---|---|---|
| Cyclomethicone: | | 10.0% |
| Inverse latex of Example 3: | | 0.8% |
| Montanov^{™} 68: | | 4.5% |
| Perfluoropolymethyl isopropyl ether: | | 0.5% |
| Preserving agent: | | 0.65% |
| Lysine: | | 0.025% |
| EDTA (disodium salt): | | 0.05% |
| Pemulen^{™} TR1: | | 0.2% |
| Glycerol: | | 3.0% |
| Water: | qs 100% | |

### Example 18: Gloss gel

| | | |
|---|---|---|
| Inverse latex of Example 2: | | 1.5% |
| Volatile silicone: | | 25.0% |
| Monopropylene glycol: | | 25.0% |
| Demineralized water: | | 10.0% |
| Glycerol: | qs | 100% |

### Example 19: Antisun cream

| | | |
|---|---|---|
| Simulsol^{™} 165: | | 3.0% |
| Montanov^{™} 202: | | 2.0% |
| C12-C15 benzoate: | | 8.0% |
| Pecosil^{™} PS 100: | | 2.0% |
| Dimethicone: | | 2.0% |
| Cyclomethicone: | | 5.0% |
| Octyl para-methoxycinnamate: | | 6.0% |
| Benzophenone-3: | | 4.0% |
| Titanium oxide: | | 8.0% |
| Xanthan gum: | | 0.2% |
| Butylene glycol: | | 5.0% |
| Demineralized water: | qs 100% | |
| Inverse latex of Example 1: | | 1.5% |
| Preserving agent, fragrance: | | qs |

### Example 20: Care gel for combination skin

| | | |
|---|---|---|
| Inverse latex of Example 3: | | 4.0% |
| Plant squalane: | | 5.0% |
| Dimethicone: | | 1.5% |
| Sepicontrol^{™} A5: | | 4.0% |
| Xanthan gum: | | 0.3% |
| Water: | qs 100% | |
| Preserving agent, fragrance: | | qs |

### Example 21: "Leave-on" protective product; anti-stress haircare

### FORMULA

| | | |
|---|---|---|
| Ketrol^{™} T: | | 0.5% |
| Mixture of cocoyl amino acids: | | 3.0% |
| Butylene glycol: | | 5.0% |
| DC 1501: | | 5.0% |
| Composition of Example 1: | | 4.0% |
| Sepicide^{™} HB: | | 0.5% |
| Sepicide^{™} CI: | | 0.3% |
| Fragrance: | | 0.3% |
| Water: | qs 100% | |

### Example 22: Antisun gel

### FORMULA

| | | |
|---|---|---|
| Inverse latex of Example 5: | | 3.00% |
| Sepicide^{™} CI: | | 0.20% |
| Sepicide^{™} HB: | | 0.30% |
| Fragrance: | | 0.10% |
| Dye: | | qs |
| Silica: | | 3.00% |
| Water: | qs 100% | |
| Silicone oil: | | 2.0% |
| Benzophenone-3: | | 5.00% |

### Example 23: Soothing antisun care (water-in-silicone)

### FORMULA

| | | |
|---|---|---|
| Inverse latex of Example 3: | | 2.00% |
| DC5225C: | | 20.00% |
| DC345: | | 10.00% |
| Sepicalm^{™} VG: | | 3.00% |
| Titanium dioxide MT100T: | | 5.00% |
| Zinc oxide Z-Cote HP1: | | 5.00% |
| Sepicide^{™} HB: | | 0.30% |
| Fragrance: | | 0.05% |
| Sepicide^{™} CI: | | 0.20% |
| Glycerol: | | 5.00% |
| Sodium chloride: | | 2.00% |
| Water: | qs 100% | |

### Example 24: self-tanning gel

| | | |
|---|---|---|
| Inverse latex of Example 2: | | 5.0% |
| Ethanol: | | 30.0% |
| Dihydroxyacetone | | 5.0% |
| Menthol: | | 0.1 % |
| Caffeine: | | 2.5% |
| Extract of ivy: | | 2.0% |
| Sepicide^{™} HB: | | 1.0% |
| Water: | qs 100% | |

The definitions of the commercial products used in the examples are as follows:
Ketrol^{™} T is xanthan gum sold by the company Kelco.
Lanol^{™} 99 is isononyl isononanoate sold by the company SEPPIC.
DC1501 is a mixture of cyclopentasiloxane and dimethiconol sold by the company Dow Chemical.
Montanov^{™} 68 (cetearyl glucoside) is a self-emulsifying composition as described in WO 92/06778, sold by the company SEPPIC.
Micropearl^{™} M 100 is an ultra-fine powder with a very soft feel and a matting action, sold by the company Matsumo.
Sepicide^{™} CI, imidazolidineurea, is a preserving agent sold by the company SEPPIC.
Pemulen^{™} TR1 is an acrylic polymer sold by Goodrich.
Simulsol^{™} 165 is self-emulsifying glyceryl stearate sold by the company SEPPIC.
Sepicide^{™} HB, which is a mixture of phenoxyethanol, methylparaben, ethylparaben, propylparaben and butylparaben, is a preserving agent sold by the company SEPPIC.
Parsol^{™} MCX is octyl para-methoxycinnamate; sold by the company Givaudan.
Lanol^{™} 37T is glyceryl triheptanoate, sold by the company SEPPIC.
Solagum^{™} L is a carrageenan sold by the company SEPPIC.
Sepicontrol^{™} A5 is a mixture of capryloylglycine, sarcosine and extract of Cinnamon zylanicum, sold by the company SEPPIC, such as those described in international patent application PCT/FR98/01313 filed on 23 June 1998.
Lanol^{™} 2681 is a coconut caprylate/caprate mixture sold by the company SEPPIC.
Montanov^{™} 202 is an APG/fatty alcohol composition as described in WO 98/47610, sold by the company SEPPIC.
DC 345 is a cyclomethicone sold by the company Dow Coming.
DC 5225C is a mixture of cyclopentasiloxane and dimethiconecopolyol sold by the company Dow Coming.
MT100VT is a micronized titanium dioxide that has undergone a surface treatment (aluminum hydroxide/stearic acid) distributed by the company Unipex.
Z-Cote HP1 is a micronized zinc oxide that has undergone a surface treatment, distributed by Gattefosse.
Pecosil^{™} PS100 is Dimethicone peg-7 phosphate commercialized by the company Phoenix.
Micropearl^{™} M305 is an ultra-fine powder of methyl methacrylate crosspolymer with a very soft feel commercialized by the company Matsumo.
Montanov^{™} S is a cocoglucosides and coconut alcohols emulsifying composition as described in WO 98/47610, commercialized by the company SEPPIC.
Dow Corning^{™} Q2 1401 is a mixture formed from a polydimethylsiloxane hydroxylated at the chain end (known as dimethiconol according to the nomenclature of the CTFA dictionary), and from a cyclic polydimethylsiloxane (known as cyclomethicone according to the nomenclature of the CTFA dictionary), sold by the company Dow Coming.
Sepifeel^{™} One is a mixture of palmitoyl praline and magnesium palmitoyl glutamate and sodium palmitoyl sarcosinate used as a texturizing agent, and sold by the company Seppic.

## Claims

1. Composition comprising an oil phase, an aqueous phase, at least one emulsifying system of water-in-oil (W/O) type, optionally at least one emulsifying system of oil-in-water (O/W) type, in the form of an inverse latex comprising from 10% to 80% by weight and preferably from 20% to 70% by weight and most preferably from 30% to 60% by weight, of a linear partially or totally salified anionic polyelectrolyte, wherein said oil phase comprises a silicone oil or a mixture of silicone oils.

2. Composition according to claim 1, wherein the anionic polyelectrolyte is a copolymer of at least one monomer having a strong acid function either, free, partially salified or totally salified with at least one neutral monomer or a copolymer of at least one monomer having at least a weak acid function either, free, partially salified or totally salified with at least one neutral monomer.

3. Composition according to claim 2, wherein the anionic polyelectrolyte is a copolymer of 2-methyl-2-[(1-oxo-2-propenyl)amino]-1-propanesulfonic acid, free, partially salified or totally salified with a neutral monomer or a copolymer of a monomer having a weak acid function either, free, partially salified or totally salified with a neutral monomer.

4. Composition according to claim 3, wherein the anionic polyelectrolyte is a copolymer of acrylic acid or of 3-methyl-3-[(1-oxo-2-propenyl)amino] butanoic acid, free, partially salified or totally salified, with a neutral monomer.

5. Composition according to one of claims 1 to 4, wherein the anionic polyelectrolyte is a copolymer of a monomer having a strong acid function either, free, partially salified or totally salified with N-tris(hydroxymethyl) methylacrylamide or a copolymer of a monomer having a weak acid function either, free, partially salified or totally salified with N-tris(hydroxymethyl) methylacrylamide,

6. Composition according to claim 5, chosen from:
- an inverse latex of a copolymer of 2-methyl-2- [(1-oxo-2-propenyl)amino]-1-propanesulfonic acid, free, partially salified or totally salified with N-tris(hydroxymethyl) methylacrylamide,
- an inverse latex of a copolymer of acrylic acid free, partially salified or totally salified, with N-tris(hydroxymethyl) methylacrylamide, and
- an inverse latex of a copolymer of 3-methyl-3-[(1-oxo-2-propenyl)amino] butanoic acid, free, partially salified or totally salified, with N-tris(hydroxymethyl) methylacrylamide.

7. Composition according to claim 1 or claim 2, wherein the anionic polyelectrolyte is a terpolymer of at least one monomer having a strong acid function either, free, partially salified or totally salified with at least one first neutral monomer different from N-tris(hydroxymethyl) methylacrylamide, and with N-tris(hydroxymethyl) methylacrylamide.

8. Composition according to claim 7chosen from:
- an inverse latex of a terpolymer of 2-methyl-2-[(1-oxo-2-propenyl)amino]-1-propanesulfonic acid, free, partially salified or totally salified, with (2-hydroxyethyl)acrylate and with N-tris(hydroxymethyl) methylacrylamide,
- an inverse latex of a terpolymer of 2-methyl-2-[(1-oxo-2-propenyl)amino]-1-propanesulfonic acid, free, partially salified or totally salified with acrylamide and with N-tris(hydroxymethyl) methylacrylamide, and
- an inverse latex of a terpolymer of 2-methyl-2-[(1-oxo-2-propenyl)amino]-1-propanesulfonic acid, free, partially salified or totally salified with N,N-dimethylacrylamide and with N-tris(hydroxymethyl) methylacrylamide,

9. Composition according to claim 1 or claim 2, wherein the anionic polyelectrolyte, is a terpolymer of at least one monomer having a weak acid function either, free, partially salified or totally salified with at least one first neutral monomer different from N-tris(hydroxymethyl) methylacrylamide, and with N-tris(hydroxymethyl) methylacrylamide.

10. Composition according to claim 9, chosen from:
- an inverse latex of a terpolymer of acrylic acid free, partially salified or totally salified with (2-hydroxyethyl) acrylate and with N-tris(hydroxymethyl) methylacrylamide,
- an inverse latex of a terpolymer of 3-methyl-3-[(1-oxo-2-propenyl)amino] butanoic acid, free, partially salified or totally salified, with (2-hydroxyethyl)acrylate and with N-tris(hydroxymethyl) methylacrylamide,
- an inverse latex of a terpolymer of acrylic acid free, partially salified or totally salified, with acrylamide and with N-tris(hydroxymethyl) methylacrylamide,
- an inverse latex of a terpolymer of 3-methyl-3-[(1-oxo-2-propenyl)amino] butanoic acid, free, partially salified or totally salified, with acrylamide and with N-tris(hydroxymethyl) methylacrylamide,
- an inverse latex of a terpolymer of acrylic acid free, partially salified or totally salified, with N,N-dimethylacrylamide and with N-tris(hydroxymethyl) methylacrylamide, and
- an inverse latex of a terpolymer of 3-methyl-3-[(1-oxo-2-propenyl)amino] butanoic acid, free, partially salified or totally salified, with N,N-dimethylacrylamide and with N-tris(hydroxymethyl) methylacrylamide,

11. Composition according to claim 1 or claim 2, wherein the anionic polyelectrolyte is a terpolymer of at least one monomer having a strong acid function either, free, partially salified or totally salified with at least one monomer having a weak acid function either, free, partially salified or totally salified and with N-tris(hydroxymethyl) methylacrylamide.

12. Composition according to claim 11, chosen from:
- an inverse latex of a terpolymer of 2-methyl-2-[(1-oxo-2-propenyl)amino]-1-propanesulfonic acid, free, partially salified or totally salified, with acrylic acid free, partially salified or totally salified, and with N-tris(hydroxymethyl) methylacrylamide, and
- an inverse latex of a terpolymer of 2-methyl-2-[(1-oxo-2-propenyl)amino]-1-propanesulfonic acid, free, partially salified or totally salified, with 3-methyl-3-[(1-oxo-2-propenyl)amino] butanoic acid, free, partially salified or totally salified, and with N-tris(hydroxymethyl) methylacrylamide.

13. Composition according to one of claims 1 to 12 which comprises an (O/W) emulsifying system.

14. Cosmetic, dermopharmaceutical or pharmaceutical composition, **characterized in that** it comprises as thickening and/or emulsifying compound, at least one inverse latex as defined in one of claims 1 to 13.
